# EUROPEAN PATENT APPLICATION

(11) **EP 1 736 146 A1**
(43) Date of publication of application: **27.12.2006**
(21) Application number: 06253183.5
(22) Date of filing: 20.06.2006
(51) Int. Cl.: A61K 9/70, A61K 31/465

(54) **Nicotine transdermal delivery system**

(30) Priority: 21.06.2005 JP 2005180785
(71) Applicant: NITTO DENKO CORPORATION, Ibaraki-shi, Osaka 567-8680 (JP)
(72) Inventor: Satoda, Shiro, Nitto Denko Corporation, Ibaraki-shi, Osaka 567-8680 (JP); Kuroda, Hidetoshi, Nitto Denko Corporation, Ibaraki-shi, Osaka 567-8680 (JP); Saito, Junichi, Nitto Denko Corporation, Ibaraki-shi, Osaka 567-8680 (JP); Ninomiya, Kazuhisa, Nitto Denko Corporation, Ibaraki-shi, Osaka 567-8680 (JP)
(74) Representative: Duckworth, Timothy John

(57) **Abstract**

The present invention provides a nicotine transdermal delivery system including an adhesive layer containing a free base nicotine and a liquid ingredient compatible with the adhesive, wherein the adhesive layer is crosslinked, and the liquid ingredient is contained in a proportion of 20-75 parts by weight, per 100 parts by weight of the adhesive layer as a whole. The nicotine transdermal delivery system has good adhesiveness and cohesion, and simultaneously achieves low irritation to the skin during peeling off and a fine feeling during adhesion.

## Description

### TECHNICAL FIELD OF THE INVENTION

The present invention relates to a nicotine transdermal delivery system to be adhered to the outer skin to allow transdermal absorption of nicotine into the body.

### BACKGROUND OF THE INVENTION

It is well known that nicotine contained in cigarettes is deeply involved in habitual smoking. As a method for reducing smoking, administration of nicotine in a form other than smoking into the living organism has been proposed to suppress habitual smoking, and various nicotine administration methods have been proposed with the growing antismoking mood in the world. These methods are called what is called a nicotine supplement therapy, which includes the following methods.

One of them is a method of administering nicotine contained in a chewing gum or drug lozenge into the body from the mouth cavity. According to this administration method, nicotine is absorbed from mucous membrane in the mouth cavity while patients chew a gum or drug lozenge in the mouth cavity. In fact, however, a large amount of nicotine is swallowed down with the saliva, by which the nicotine is mostly metabolized and cleared from the blood during passage through the liver as in the case of oral administration of general drugs, and a high effect cannot be expected. Moreover, since this method is a temporary administration method, frequent application is necessary and, since nicotine directly touches the inner wall of the mouth and esophagus, uncomfortable side effects such as bad taste, heartburn, nausea, hiccup and the like are caused.

There is a method wherein a nicotine-containing solution is placed in a plastic one time container or multiple-time use container, which is then inserted into a nostril for direct administration of the nicotine solution in the container from the nasal mucous membrane. However, this method is not preferable from a hygiene standpoint, since the container directly contacts the nasal mucous membrane. In addition, handling and management is difficult. Moreover, since only a temporary effect is expected as in the above method, frequent administration is necessary. In particular, this method is problematic since it includes insertion of the container into the nostril, which makes administration in front of others embarrassing, and the like.

As a method that has solved the problems of the above-mentioned two administration methods, a method comprising transdermally administering nicotine by a nicotine transdermal delivery system has been put into practice in recent years (e.g., US Patent No. 4597961). The method using a nicotine transdermal delivery system can maintain nicotine blood concentration at a constant level for a long time by one time adhesion and is free of uncomfortable side effects associated with the method comprising administration in the mouth cavity. In addition, since it is easy in the handling and the like and highly convenient for use, a method for continuous administration of nicotine by transdermal absorption has been mainly employed.

For use of these nicotine transdermal delivery systems, a stop-smoking program has been set to quit smoking, and the program generally requires once-a-day adhesion for several weeks according to the program. While the patch size is changed to gradually reduce the daily dose of nicotine, it generally takes 8 to 10 weeks at maximum before the end of the program. During the period, the patients are required to exchange the nicotine transdermal delivery system every day.

In such use of a nicotine transdermal delivery system, nicotine transdermal delivery systems using a conventional acrylic adhesive or rubber adhesive as an adhesive in an adhesive layer to fix the preparation to the skin are associated with a problem in that skin irritation occurs during peeling of the preparation for exchange. In addition, for convenience of the production method of the preparation, a non-woven fabric or paper is inserted into the adhesive layer of the preparation, making the whole preparation thicker, which in turn causes a rough feeling during application due to physical stimulation and a feeling during adhesion is not necessarily good.

As used herein, a good feeling during adhesion means that, in general, an adhesive preparation is superior in the fixedness to the skin, a soft feeling and the like, and the patients do not feel a foreign sensation near the preparation adhesion site. A bad feeling during adhesion means that, in general, an adhesive preparation is inferior in the fixedness to the skin, a soft feeling and the like, and the patients feel a foreign sensation (rough feeling etc.) near the preparation adhesion site.

While normal rubber adhesives adhere well to the dry skin, since adhesives have low hydrophilicity, sweat is pooled in the interface between the skin and the adhesion surface during application, which may lift the adhesive and cause delamination, thus resulting in falling off during use. Furthermore, the sweat develops stuffiness to easily cause irritation, and a feeling during adhesion is not necessarily good.

In the case of polyisobutylene (PIB) adhesive, which is a representative rubber adhesive, there is available a technique including mixing a high molecular weight component and a low molecular weight component to impart good adhesiveness and cohesion to the human skin (e.g., JP-B-3035346). However, to achieve good skin adhesion, cohesion needs to be sacrificed somewhat and, when adhesiveness is preferentially considered, a problem occurs in that an adhesive flows out from the edge of a preparation during preservation due to the decreased cohesion, thus causing a cold flow (low temperature flow). The cold flow invites difficulty in taking out the preparation from the packaging material due to the attachment of adhesive in the packaging material. Particularly, since nicotine has a strong plasticizing action on the adhesive, the above-mentioned cold flow phenomenon remarkably expresses in a nicotine transdermal delivery system.

As mentioned above, a nicotine transdermal delivery system having an adhesive layer that exhibits good adhesiveness and cohesion has not been known.

In view of the above-mentioned situation, for use of a nicotine transdermal delivery system, it is currently considered that the adhesion site is desirably changed every time the preparation is exchanged, thereby to prevent skin irritation during application. However, since the preparation is exchanged to a new one, the stimulation caused by peeling off cannot be ignored.

Therefore, the development of a nicotine transdermal delivery system having fine adhesiveness and cohesion and simultaneously showing low irritation to the skin during peeling off and a fine feeling during adhesion is desired.

Moreover, since nicotine is a highly volatile and highly toxic drug, production methods of various nicotine transdermal delivery systems taking into consideration such properties are known. There is known a method comprising immersing a highly volatile nicotine compound in an absorptive material such as a non-woven fabric, and sandwiching the material with adhesive layers (e.g., JP-B-2708391). According to this production method, a non-woven fabric is placed between adhesive layers. Since the non-woven fabric is an element used for the convenience of a step, which does not function as a preparation, the preparation gains thickness comparable to the non-woven fabric sandwiched between adhesive layers, which in turn deprives the obtained preparation of a soft feeling and causes an adverse influence on the feeling during adhesion.

Furthermore, a production method is known, which comprises preparing an adhesive coating solution by dissolving a silicone adhesive in a solvent having a low boiling point, such as hexane and the like, and applying the solution at a low temperature to decrease volatilization of nicotine as much as possible (e.g., JP-A-2002-531488). However, low temperature coating according to this method cannot completely solve the problem of nicotine volatilization, and an operation to charge an additional amount is necessary to achieve a desired amount of nicotine. Moreover, the production method is highly complicated due to the special constitution of the method, and the obtained transdermal absorption preparation is inferior in the adhesiveness.

### DISCLOSURE OF THE INVENTION

The present invention aims at provision of a nicotine transdermal delivery system having good adhesiveness and cohesion, and simultaneously meeting low irritation to the skin during peeling off and a fine feeling during adhesion.

The present inventors have conducted intensive studies in an attempt to solve the aforementioned problems and found that use of a crosslinked adhesive layer containing a free base nicotine and a liquid ingredient compatible with an adhesive in the below-mentioned particular proportion as a nicotine transdermal delivery system affords good adhesiveness and cohesion, and simultaneously achieves low irritation to the skin and a fine feeling during adhesion, which resulted in the completion of the present invention.

Accordingly, the present invention provides the following.
(1) A nicotine transdermal delivery system comprising an adhesive layer comprising a free base nicotine and a liquid ingredient compatible with the adhesive, wherein the adhesive layer is crosslinked, and the liquid ingredient is contained in a proportion of 20-75 parts by weight, per 100 parts by weight of the adhesive layer as a whole.
(2) The nicotine transdermal delivery system of the above-mentioned (1), wherein the aforementioned liquid ingredient is fatty acid alkyl ester and/or glycerol fatty acid ester.
(3) The nicotine transdermal delivery system of the above-mentioned (1) or (2), wherein the aforementioned liquid ingredient is contained in a proportion of 20-60 parts by weight, per 100 parts by weight of the adhesive layer as a whole.
(4) The nicotine transdermal delivery system of the above-mentioned (1) or (3), wherein the aforementioned liquid ingredient is isopropyl myristate and/or caprylic • capric triglyceride.
(5) The nicotine transdermal delivery system of any of the above-mentioned (1)-(4), wherein the adhesive contained in the adhesive layer is an acrylic adhesive.
(6) The nicotine transdermal delivery system of the above-mentioned (5), wherein the acrylic adhesive comprises (meth)acrylic acid alkyl ester and a vinyl monomer having a functional group capable of being involved in a crosslinking reaction in a weight ratio of 40-99.9:0.1-10.
(7) The nicotine transdermal delivery system of the above-mentioned (6), wherein the (meth)acrylic acid alkyl ester is 2-ethylhexyl acrylate and the vinyl monomer having a functional group capable of being involved in a crosslinking reaction is acrylic acid and/or 2-hydroxyethyl acrylate.
(8) The nicotine transdermal delivery system of any of the above-mentioned (1)-(7), wherein the adhesive layer comprises the free base nicotine in a proportion of 1-40 wt%.

### BRIEF DESCRIPTION OF THE DRAWINGS

Fig. 1 is a graph showing the results of the shed snakeskin permeability test (Flux) in Experimental Example 3 using the preparations of Examples 1-5 and control.
Fig. 2 is a graph showing the results of the shed snakeskin permeability test (Flux) in Experimental Example 3 using the preparations of Examples 6-10 and control.
Fig. 3 is a graph showing the results of the shed snakeskin permeability test (cumulative permeation amount of drug) in Experimental Example 3 using the preparations of Examples 1-5 and control.
Fig. 4 is a graph showing the results of the shed snakeskin permeability test (cumulative permeation amount of drug) in Experimental Example 3 using the preparations of Examples 6-10 and control.
Fig. 5 is a graph showing the results of the hairless mouse skin permeability test (Flux) in Experimental Example 4 using the preparations of Examples 11-14 and control.
Fig. 6 is a graph showing the results of the hairless mouse skin permeability test (cumulative permeation amount of drug) in Experimental Example 4 using the preparations of Examples 11-14 and control.
Fig. 7 is a graph showing the results of the hairless mouse skin permeability test (Flux) in Experimental Example 5 using the preparations of Examples 15-17 and control.
Fig. 8 is a graph showing the results of the hairless mouse skin permeability test (cumulative permeation amount of drug) in Experimental Example 5 using the preparations of Examples 15-17 and control.
Fig. 9 is a graph showing the results of the hairless mouse skin permeability test (Flux) in Experimental Example 6 using the preparations of Example 18 and control.
Fig. 10 is a graph showing the results of the hairless mouse skin permeability test (Flux) in Experimental Example 6 using the preparations of Examples 19-22.
Fig. 11 is a graph showing the results of the hairless mouse skin permeability test (cumulative permeation amount of drug) in Experimental Example 6 using the preparations of Examples 19-22.

### EFFECTS OF THE INVENTION

Since the nicotine transdermal delivery system of the present invention contains, in an adhesive layer, a free base nicotine and a liquid ingredient compatible with the adhesive in a particular range of concentration and the adhesive layer is subjected to a crosslinking treatment, it shows good adhesiveness and cohesion, and superior fixedness and a soft feeling during application, as well as less skin irritation during peeling off of the preparation. Therefore, the preparation can be used comfortably for a long time for use accompanying daily exchange of the preparation.

### BEST MODE FOR EMBODYING THE INVENTION

The nicotine transdermal delivery system of the present invention characteristically contains, in an adhesive layer, a free base nicotine and a liquid ingredient compatible with the adhesive in the adhesive layer, wherein the adhesive layer is crosslinked and the liquid ingredient is contained in a proportion of 20-75 parts by weight, per 100 parts by weight of the adhesive layer as a whole.

In the present invention, a free base nicotine (i.e., free form of nicotine, without forming a salt) is used since transdermal absorbability is high, is liquid at ambient temperature and can be directly applied. While the content of a free base nicotine can be appropriately determined according to the administration object, it is generally contained in an adhesive layer in a proportion of about 1-40 wt%, preferably 5-30 wt%. When the content is less than 1 wt%, the release in an amount effective for the treatment cannot be expected and, when it exceeds 40 wt%, the treatment effect may be limited and economical disadvantages may be caused.

In the present invention, the adhesive layer contains a liquid ingredient compatible with the adhesive in the adhesive layer. Addition of the liquid ingredient aims at imparting a soft feeling by plasticizing the adhesive, and reducing pain and skin irritation caused by adhesion to the skin when peeling off the nicotine transdermal delivery system from the skin. Therefore, the liquid ingredient is not particularly limited as long as it is compatible with the adhesive and has a plasticizing action, and one having an absorption promoting action to improve the transdermal absorbability of the free base nicotine is preferably used. As the liquid ingredient, for example, fats and oils such as olive oil, castor oil, squalene, lanoline and the like; organic solvents such as dimethyldecyl sulfoxide, methyloctyl sulfoxide, dimethyl sulfoxide, dimethylformamide, dimethylacetamide, dimethyllaurylamide, methylpyrrolidone, dodecylpyrrolidone and the like; liquid surfactants; diisopropyl adipate, phthalic acid (di)ester (e.g., diisononyl phthalate, di(2-ethylhexyl) phthalate and the like), plasticizers such as diethyl sebacate and the like; hydrocarbons such as liquid paraffin; fatty acid esters such as fatty acid alkyl ester (e.g., alcohol wherein the alkyl moiety is linear, branched chain or cyclic alkyl having 1 to 13 carbon atoms, ester with saturated or unsaturated fatty acid having 8 to 18 carbon atoms and the like, specifically, ethyl oleate, isopropyl palmitate, octyl palmitate, isopropyl myristate, isotridecyl myristate, ethyl laurate and the like), glycerol fatty acid ester (e.g., ester of glycerol and saturated or unsaturated fatty acid having 8 to 16 carbon atoms and the like, specifically, caprylic · capric triglyceride and the like), propylene glycol fatty acid ester (e.g., ester of propylene glycol and saturated or unsaturated fatty acid having 8 to 16 carbon atoms, and the like, specifically, propylene glycol dicaprylate and the like), pyrrolidonecarboxylic acid alkyl ester and the like; aliphatic dicarboxylic acid alkyl ester (e.g., ester of alcohol wherein the alkyl moiety is a linear, branched chain or cyclic alkyl having 1 to 4 carbon atoms and saturated or unsaturated aliphatic dicarboxylic acid having 6 to 16 carbon atoms, and the like, specifically, diisopropyl adipate, diethyl sebacate and the like); silicone oil; ethoxylated stearyl alcohol and the like can be mentioned. Of these, one or more kinds are used in combination. Of the above-mentioned, fatty acid alkyl ester, glycerol fatty acid ester, propylene glycol fatty acid ester and aliphatic dicarboxylic acid alkyl ester exemplified above are preferable, and fatty acid alkyl ester and glycerol fatty acid ester are particularly preferable, particularly from the viewpoints of low skin irritation, safety and easy availability. To be specific, diisopropyl myristate, caprylic capric triglyceride, isopropyl palmitate, diethyl sebacate and propylene glycol dicaprylate are particularly preferable, and isopropyl myristate and caprylic · capric triglyceride are more preferable.

The caprylic · capric triglyceride is a triester of caprylic acid and capric acid, and glycerol. In the present invention, while the ratio of caprylic acid to capric acid is not particularly limited, caprylic acid:capric acid is generally about 5:5- about 9:1 (weight ratio). The caprylic • capric triglyceride may be a commercially available product (e.g., Coconad MT (manufactured by Kao Corporation) and the like).

As the liquid ingredient, fatty acid alkyl ester, especially isopropyl myristate, is preferable particularly from the aspect of good transdermal absorbability. Particularly, from the aspect of good adhesion, glycerol fatty acid ester, especially caprylic • capric triglyceride, is preferable. Particularly, good adhesion can be afforded and appropriate transdermal absorbability, which is not too high or too low, can be afforded, for example, in the aforementioned stop-smoking program (especially, a one time/day adhesion program), as a result of which the nicotine blood concentration can be maintained at a constant level for a long time by one time adhesion. From such aspect, a system including coexistence of isopropyl myristate and caprylic • capric triglyceride is preferable.

While the content ratio of isopropyl myristate and caprylic capric triglyceride, when they are coexistent, is not particularly limited, from the aspect of achieving appropriate transdermal absorbability and good adhesion, it is particularly isopropyl myristate:caprylic • capric triglyceride=about 1:8-2:1 (weight ratio).

The mixing ratio (content ratio) of the liquid ingredient is 20-75 parts by weight, preferably 20-60 parts by weight, per 100 parts by weight of the adhesive layer as a whole and, from the aspect of low skin irritation, it is particularly preferably 25-55 parts by weight and more preferably 30-50 parts by weight, per 100 parts by weight of the adhesive layer as a whole. When the content ratio of the liquid ingredient is less than 20 parts by weight, the adhesive force becomes too strong and skin irritation easily occurs when the preparation is peeled off from the skin surface. When it exceeds 75 parts by weight, the adhesive force becomes too weak to highly likely result in falling of the preparation from the skin surface during application.

The adhesive to form an adhesive layer to be used in the present invention is not particularly limited as long as it can be crosslinked and, for example, rubber polymer materials such as silicone rubber, polyisoprene rubber, polyisobutylene rubber, styrene-butadiene rubber, styrene-isoprene-styrene block copolymer rubber, styrene-butadiene-styrene block copolymer rubber and the like, vinyl polymer materials such as polyvinyl alcohol, polyvinyl alkyl ether, polyvinyl acetate and the like, and the below-mentioned acrylic adhesive containing (meth)acrylic acid alkyl ester as a main component can be mentioned. As the adhesive to be used in the present invention, an acrylic adhesive (particularly, acrylic adhesive containing (meth)acrylic acid alkyl ester as a main component) is preferable.

The crosslinking treatment is conducted by a chemical crosslinking treatment, a physical crosslinking treatment and the like. Of these, a crosslinking treatment by electron beam irradiation or UV irradiation as a physical crosslinking treatment, or a crosslinking treatment by addition of a crosslinking agent as a chemical crosslinking treatment is preferable. In addition, the above-mentioned adhesive preferably contains a functional group capable of being involved in a crosslinking reaction, such as hydroxyl group, carboxyl group, vinyl group and the like.

Specific examples of the adhesive containing a functional group capable of being involved in a crosslinking reaction are shown below. For example, the adhesive is crosslinked by a general method comprising, during synthesis of a polymer to be the adhesive, copolymerizing monomers having a hydroxyl group such as hydroxyethyl (meth)acrylate and the like, or monomers having a carboxyl group such as acrylic acid, maleic acid and the like and reacting with a crosslinking agent or introducing a reaction point for crosslinking by radiation, or adding a monomer having two or more vinyl groups such as divinylbenzene, ethylene glycol dimethacrylate and the like and copolymerizing the monomer and other monomers, thereby causing crosslinking during copolymerization reaction between molecules or within molecules and the like.

As the adhesive containing a functional group capable of being involved in a crosslinking reaction, an acrylic adhesive containing a functional group capable of being involved in a crosslinking reaction is preferable, since a crosslinking treatment can be easily conducted.

As a specific example of the adhesive containing a functional group capable of being involved in a crosslinking reaction, an acrylic adhesive containing a functional group capable of being involved in a crosslinking reaction is explained in detail in the following.

As such acrylic adhesive, a copolymer composition containing a copolymer of (meth)acrylic acid alkyl ester to be the main component, a vinyl monomer having a functional group capable of being involved in a crosslinking reaction as a second monomer component and, where necessary, a tertiary monomer component is preferable from the aspect of an easy crosslinking treatment.

Examples of (meth)acrylic acid alkyl ester, (meth)acrylic acid alkyl ester wherein the alkyl group is linear, branched chain or cyclic alkyl group having 1 to 18 carbon atoms (e.g., methyl, ethyl, propyl, butyl, pentyl, hexyl, cyclohexyl, heptyl, octyl, 2-ethylhexyl, nonyl, decyl, undecyl, dodecyl, tridecyl etc.) and the like can be mentioned, with preference given to (meth)acrylic acid alkyl ester wherein the alkyl group is linear, branched chain or cyclic alkyl group having 4 to 18 carbon atoms (e.g., butyl, pentyl, hexyl, cyclohexyl, heptyl, octyl, 2-ethylhexyl, nonyl, decyl, undecyl, dodecyl, tridecyl and the like). These (meth)acrylic acid alkyl esters can be used in combination of one or more kinds thereof.

To impart the adhesiveness at ambient temperature, a monomer that lowers the glass transition temperature of the polymer is more preferable. Thus, (meth)acrylic acid alkyl ester wherein the alkyl group is a linear, branched chain or cyclic alkyl group having 4 to 8 carbon atoms (e.g., butyl, pentyl, hexyl, cyclohexyl, heptyl, octyl, 2-ethylhexyl and the like, preferably, butyl, 2-ethylhexyl, cyclohexyl, particularly preferably 2-ethylhexyl) is more preferable. Specifically, butyl acrylate, 2-ethylhexyl acrylate, 2-ethylhexyl methacrylate, cyclohexyl acrylate, cyclohexyl methacrylate and the like are more preferable, and 2-ethylhexyl acrylate is most preferable.

As the second monomer component, a vinyl monomer having a functional group capable of being involved in a crosslinking reaction is used. As examples of the functional group capable of being involved in a crosslinking reaction, hydroxyl group, carboxyl group, vinyl group and the like can be mentioned, with preference given to hydroxyl group and carboxyl group. As the vinyl monomer having a functional group capable of being involved in a crosslinking reaction, specifically, hydroxyethyl (meth)acrylate, hydroxypropyl (meth)acrylate, (meth)acrylic acid, itaconic acid, maleic acid, methaconic acid, citraconic acid, glutaconic acid and the like can be mentioned. Of these, acrylic acid, methacrylic acid and hydroxyethyl acrylate (particularly, 2-hydroxyethyl acrylate) are preferable from the aspect of easy availability. These second monomer components can be used in combination of one or more kinds thereof.

A tertiary monomer component may be used for adjusting cohesion of the adhesive layer, adjusting solubility • releasability of the free base nicotine. As the tertiary monomer component, for example, vinyl esters such as vinyl acetate, vinyl propionate and the like; vinyl ethers such as methyl vinyl ether, ethyl vinyl ether and the like; vinyl amides such as N-vinyl-2-pyrrolidone, N-vinyl caprolactam and the like; alkoxyl group-containing monomers such as (meth)acrylic acid methoxyethyl ester, (meth)acrylic acid ethoxyethyl ester and the like; hydroxyl group-containing monomers such as hydroxypropyl (meth)acrylate, α-hydroxymethyl acrylate and the like (not taken as a crosslinking point due to the use as a tertiary monomer component); amido group-containing monomers such as (meth)acrylamide, dimethyl(meth)acrylamide and the like; vinyl monomers such as styrene, vinyl pyridine, vinyl imidazole, vinyl morpholine and the like; and the like can be mentioned. These tertiary monomer components can be used in combination of one or more kinds thereof.

The acrylic adhesive is preferably obtained by copolymerization of (meth)acrylic acid alkyl ester and the second monomer (a vinyl monomer having a functional group capable of being involved in a crosslinking reaction) at a weight ratio of (meth)acrylic acid alkyl ester:second monomer=40-99.9:0.1-10.

When the tertiary monomer component is contained as necessary, the acrylic adhesive is preferably copolymerized by adding (meth)acrylic acid alkyl ester, the second monomer and the tertiary monomer at a weight ratio of (meth)acrylic acid alkyl ester:second monomer:tertiary monomer=40-99.9:0.1-10:0-50, more preferably at a weight ratio of 60-95:3-5:15-30.

The polymerization reaction is not particularly limited and can be carried out according to a method known per se. For example, a method comprising reacting the above-mentioned monomer by addition of a polymerization initiator (e.g., benzoyl peroxide, azobisisobutyronitrile and the like) in a solvent (e.g., ethyl acetate and the like) at 50-70°C for 5-48 hr can be mentioned.

In the present invention, 2-ethylhexyl acrylate as (meth)acrylic acid alkyl ester and acrylic acid and/or 2-hydroxyethyl acrylate as the second monomer are preferably used in combination.

In the present invention, a mixture of the above-mentioned adhesive and the liquid ingredient is subjected to a crosslinking treatment to afford suitable cohesion for application to the human skin. The crosslinking treatment can be conducted by a chemical crosslinking treatment using a crosslinking agent, a physical crosslinking treatment using electron beam (e.g., γ ray) irradiation, UV irradiation and the like, and the like. The crosslinking treatment can be conducted according to a method generally employed in the pertinent field.

As the crosslinking agent to be used for chemical crosslinking treatment, isocyanate compounds (e.g., Coronate HL (product name, manufactured by Nippon Polyurethane Industry Co., Ltd.) and the like), metal chelate compounds (e.g., metal chelate compounds made of titanium, zirconium, zinc or aluminum, specifically aluminum ethylacetoacetate• diisopropylate (e.g., ALCH (product name, manufactured by Kawaken Fine Chemicals Co., Ltd.) and the like)), organic peroxide, epoxy compound, melamine resin, metal alcoholate and the like can be mentioned. From the aspect of reactivity and handling property, isocyanate compounds (e.g., Coronate HL (product name, manufactured by Nippon Polyurethane Industry Co., Ltd.) and the like); a metal chelate compound made of titanium, zirconium, zinc or aluminum, specifically aluminum ethylacetoacetate • diisopropylate (e.g., ALCH (product name, manufactured by Kawaken Fine Chemicals Co., Ltd.) and the like) are preferable. These crosslinking agents do not cause thickening of the solution up to coating and drying, and is extremely superior in the workability.

The content of the crosslinking agent is about 0.01 - 5 parts by weight, per 100 parts by weight of the adhesive. When it is less than 0.01 part by weight, the crosslinking points may be too few, sufficient cohesion cannot be imparted to the adhesive layer, and adhesive residue and strong skin irritation may be expressed due to a cohesive failure upon peeling off. When it is greater than 5 parts by weight, cohesion becomes high but sufficient skin adhesion may not be achieved, possibly causing skin irritation and decomposition of a free base nicotine, due to the residual unreacted initiator.

The chemical crosslinking treatment can be conducted by, after addition of a crosslinking agent, heating to a temperature not less than crosslinking reaction temperature. The heating temperature can be appropriately determined according to the kind of the crosslinking agent. Since the production step can be simplified when a crosslinking reaction occurs in parallel with drying during heating for a drying step, the heating temperature is preferably 50°C to 140°C, more preferably 60°C to 100°C. The heating time is preferably one day to one week, more preferably one day to three days.

The thickness of the adhesive layer in the nicotine transdermal delivery system of the present invention is not particularly limited but is generally 40-300 µm, preferably 50-200 µm.

The nicotine transdermal delivery system of the present invention generally has a support, an adhesive layer and a release liner. That is, the nicotine transdermal delivery system of the present invention has a structure where the aforementioned adhesive layer is laminated on the support, and the adhesive surface of the adhesive layer (surface of the adhesive layer opposite from where the support is laminated) is preferably protected by being covered with a release liner until immediately before use. In addition, a back coating agent such as silicone, fluorine, wax and the like may be applied on a support to give a roll without using a release liner.

While the support is not particularly limited and any known support can be used, the free base nicotine contained in the adhesive layer is preferably not lost from the back through the support to cause low content. Accordingly, the support is preferably made from a material impermeable to a free base nicotine. Specifically, a single film of polyester, nylon, saran, polyethylene, polypropylene, ethylene-vinyl acetate copolymer, polyvinyl chloride, ethylene-ethyl acrylate copolymer, polytetrafluoroethylene, metal foil, polyethylene terephthalate and the like, a laminate film wherein one or more kinds thereof are laminated and the like can be used. To improve adhesion (anchor property) between the support and the adhesive layer, the support is, from among these, preferably a laminate sheet of a non-porous sheet made from the above-mentioned material and the following porous sheet, and the adhesive layer is preferably formed on the porous sheet side.

The porous sheet is not particularly limited as long as the anchor property with the adhesive layer can be improved and, for example, paper, woven fabric, non-woven fabric (e.g., polyester non-woven fabric, polyethylene terephthalate non-woven fabric and the like), a sheet obtained by a mechanical perforation treatment of the above-mentioned film (e.g., a single film of polyester, nylon, saran, polyethylene, polypropylene, ethylene-vinyl acetate copolymer, polyvinyl chloride, ethylene-ethyl acrylate copolymer, polytetrafluoroethylene, metal foil, polyethylene terephthalate and the like, a laminate film wherein one or more kinds thereof are laminated and the like), and the like can be mentioned. Particularly, paper, woven fabric, non-woven fabric (e.g., polyester non-woven fabric, polyethylene terephthalate non-woven fabric and the like) are preferable. The thickness of the porous sheet is generally within the range of 10-500 µm, in consideration of the improvement of anchor property and flexibility of the adhesive layer. When a woven fabric or a non-woven fabric is used as a porous sheet, the amount of the fabric weight is preferably 5-50 g/m², preferably 8-40 g/m², for the improvement of anchor property. As the laminate sheet of a non-porous sheet and a porous sheet, a laminate sheet of a polyethylene terephthalate film and a polyester non-woven fabric or polyethylene terephthalate non-woven fabric, and the like can be mentioned.

While the thickness of the support of the nicotine transdermal delivery system of the present invention is not particularly limited, it is generally 10-500 µm, preferably 10-200 µm.

The release liner is not particularly limited, and any known release liner can be used. Specifically, as the release liner, a release liner wherein a release agent layer comprising a release agent is formed on the surface of a substrate for a release liner, a plastic film having high release property in itself, a release liner having a constitution where the aforementioned plastic film material having high release property is formed on the surface of a substrate for a release liner, and the like can be mentioned. The release surface of the release liner may be only one surface or both surfaces of the substrate.

In such a release liner, the peel treatment agent is not particularly limited and, for example, release agents such as a long chain alkyl group-containing polymer, a silicone polymer (silicone release agent), a fluorine polymer (fluorine release agent) and the like can be mentioned.

As the substrate for the release liner, for example, plastic films such as a polyethylene terephthalate film, a polyimide film, a polypropylene film, a polyethylene film, a polycarbonate film, a polyester film and the like and metal vapor deposition plastic film wherein a metal is vapor deposited on such film; papers such as Japanese paper, foreign paper, craft paper, glassine, quality paper and the like; a substrate made of a fiber material such as non-woven fabric, cloth and the like; a metal foil and the like can be mentioned.

As the plastic film having high release property in itself, for example, ethylene-α-olefin copolymers (block copolymer or random copolymer) such as polyethylene (low density polyethylene, linear low density polyethylene etc.), polypropylene, ethylene-propylene copolymer and the like, a polyolefin film made from a polyolefin resin which is a mixture thereof; Teflon (trademark) film and the like can be used.

The release layer to be formed on the surface of a substrate for the aforementioned release liner can be formed by laminating or coating the aforementioned plastic film material having high release property on the substrate for the aforementioned release liner.

The thickness (whole thickness) of the release liner is not particularly limited and, for example, can be selected from the range of not less than 15 µm (preferably 25-500 µm).

The nicotine transdermal delivery system of the present invention can be produced, for example, by the following method and the method is characterized in that a free base nicotine is contained in a previously formed, crosslinked adhesive layer (crosslinked adhesive layer) by bringing a free base nicotine into direct contact with the adhesive layer.

The free base nicotine is highly toxic and highly volatile drug, which is difficult to be dried by heating. According to this method, since a free base nicotine alone is applied to an adhesive layer after the adhesive layer forming step that requires heating, drying by heating is not necessary after the coating, and volatilization of the free base nicotine does not need to be worried about.

An adhesive, a liquid ingredient compatible with the adhesive, and a crosslinking agent (when chemical crosslinking treatment is applied) are added, a mixed solution thereof is thoroughly stirred, applied on the support and/or a release liner, and dried to give an adhesive layer having a support and/or an adhesive layer having a release liner. The drying temperature is generally 40°C to 120°C, preferably 60°C to 100°C, and the drying time is generally 3-30 min, preferably 10-15 min.

Here, an adhesive layer having a support and/or an adhesive layer having a release liner may be formed by applying the above-mentioned mixed solution on a release liner, drying the solution, adhering the support and/or the release liner to the adhesive surface of the adhesive layer thus formed.

Thereafter, a crosslinking treatment by electron beam irradiation, UV irradiation, or, when a crosslinking agent is added to the aforementioned mixed solution, heating and the like is conducted to give a crosslinked adhesive layer. When the crosslinking is conducted by a chemical crosslinking treatment, a crosslinking agent that requires a temperature not higher than the drying temperature for the crosslinking reaction is used to simultaneously conduct the crosslinking treatment with drying in the drying step. In this case, after the drying step, further heating may be employed to increase the crosslinking degree. The temperature for the further heating is generally 50-140°C, and the heating time is generally one day to one week.

After forming the crosslinked adhesive layer, a free base nicotine (liquid) is brought into direct contact with the crosslinked adhesive layer by coating, impregnation and the like. A preferable embodiment is direct coating of a crosslinked adhesive layer with a free base nicotine (liquid).

For coating, a known technique for thin film coating with a liquid can be used, since the free base nicotine has the same viscosity with water. As the known technique for thin film coating with a liquid, a method particularly used in the field of printing can be mentioned. In the event the viscosity needs to be adjusted when a method used in the field of printing is employed, an additive may be used to the extent that transdermal absorbability and adhesive performance are not affected.

Examples of the method used in the field of printing include a method comprising direct dropwise addition, a method using a gravure coater, flexo coater, calendar coater, spray coater, curtain coater, fountain coater, die coater or slit die coater, inkjet and the like. These methods can be adapted to thin film coating that general requires precision, and when the content uniformity of a drug is required as in the present invention, a coating method having high coating precision is advantageously employed. Moreover, since a free base nicotine is used as it is as a coating solution at this time, a coating method, wherein even a coating solution having a low viscosity can afford a high precision coating, is preferable. From such aspect, a method using a gravure coater or a flexo coater is preferable. A printing method can easily perform pattern coating on the surface of an adhesive layer, and is economically advantageous.

After coating, when a free base nicotine-coated crosslinked adhesive layer has a support, the adhesive surface of the crosslinked adhesive layer is adhered to and the adhesive surface of a crosslinked adhesive layer having a release liner or a release liner to give the nicotine transdermal delivery system of the present invention. When a free base nicotine-coated crosslinked adhesive layer has a release liner, the adhesive surface of the crosslinked adhesive layer and a support or the adhesive surface of a crosslinked adhesive layer having a support to give the nicotine transdermal delivery system of the present invention. The nicotine transdermal delivery system of the present invention can also be produced by adhering the adhesive surface of a free base nicotine-coated crosslinked adhesive layer having a support to the adhesive surface of a free base nicotine-coated crosslinked adhesive layer having a release liner.

Of these, a method comprising applying a free base nicotine to the adhesive surface of a crosslinked adhesive layer formed on a support and adhering a release liner to the adhesive surface, or a method comprising applying a free base nicotine to one of or both of the adhesive surface of a crosslinked adhesive layer formed on a release liner and the adhesive surface of a crosslinked adhesive layer formed on a support, and adhering these adhesive surfaces to each other is preferable.

Since an adhesive layer is subjected to a crosslinking treatment in advance, it shows lower adhesiveness to a support. Therefore, application of a free base nicotine to the adhesive surface of a crosslinked adhesive layer formed on a release liner, followed by adhesion to a support, is not preferable in view of the possibility of markedly affecting the anchor property between the adhesive layer and the support.

The shape and the size of the nicotine transdermal delivery system of the present invention are not particularly limited, and any shape and size can be employed according to the adhesion site and the like. The shape includes, for example, tape, sheet and the like. The size of the preparation is, for example, 5-30 cm².

It is preferable to cover the adhesive surface of the adhesive layer with a release liner for protection of the nicotine transdermal delivery system of the present invention until immediately before adhesion to the skin. When in use, the release liner is peeled off to expose the adhesive surface, which is then adhered to the adhesion site for the administration of the free base nicotine.

The nicotine transdermal delivery system of the present invention can be used for a nicotine supplement therapy and the like of smokers (particularly those wishing to quit smoking), according to a stop-smoking program conventionally practiced or to be practiced in the future, which aims at suppressing habitual smoking.

While the dose of a free base nicotine by the nicotine transdermal delivery system of the present invention varies depending on the age and body weight of the patients, severity of disease and the like, a transdermal absorption preparation containing 5-120 mg of a free base nicotine is generally adhered to the skin (5-30 cm²) of an adult once or so per 0.5 to 2 days.

### Examples

The present invention is explained in detail in the following by referring to Examples, which are not to be construed as limitative. Unless otherwise specified, part and % mean parts by weight and wt%, respectively, in the following.

### (Examples 1-5)

Under a nitrogen atmosphere, 2-ethylhexyl acrylate (95 parts), acrylic acid (5 parts), ethyl acetate (100 parts) and benzoyl peroxide (0.2 part, BPO, manufactured by NOF Corporation, product name Nyper BW) were polymerized in a separable flask equipped with a refluxing condenser, a stirrer, a thermometer, a dropping funnel and a nitrogen inlet tube at 60°C for 15 hr to give an adhesive solution (hereinafter to be referred to as adhesive solution A). The obtained adhesive solution A was measured out in the amounts corresponding to adhesive solid contents of 49.93, 54.923, 59.916, 64.909 and 69.902 parts and placed in respective reaction containers. Isopropyl myristate was added to each reaction container in 50, 45, 40, 35 and 30 parts relative to the adhesive solid content, Coronate HL (manufactured by Nippon Polyurethane Industry Co., Ltd.) was added as a crosslinking agent in a proportion of 0.07, 0.077, 0.084, 0.091 and 0.098 parts, respectively (0.14% of adhesive), and the mixture was thoroughly stirred. The obtained solutions were each used for the preparation of the crosslinked adhesive layers of Examples 1-5 to be mentioned below.

The obtained solution was applied to a peel treated surface of a polyester film release liner having the peel treated surface on one side to a thickness after drying of 120 µm, and dried at 100°C for 3 min to give an adhesive layer. The adhesive surface of the adhesive layer thus formed was adhered to the surface on a non-woven fabric side of a support prepared by laminating a 2 µm thick polyethylene terephthalate film on the polyester non-woven fabric (fabric weight amount 12 g/m²) by extrusion forming to give a laminate (hereinafter to be referred to as laminate 1). Separately, a peel-treated surface of a peel-treated paper release liner is adhered to the adhesive surface of the adhesive layer formed on the above-mentioned release liner in the same manner as above to give a laminate (hereinafter to be referred to as laminate 2). The laminate 1 and laminate 2 were tightly sealed, left standing at 60°C for 48 hr to form a crosslinked adhesive layer. Thereafter, the laminate 2 (paper release liner) was peeled off to expose the adhesive surface, an engraving roller (coated amount: calculated 1.8 mg/cm²) was set in a flexo printing coater (manufactured by RK Print Coat Instruments Ltd., product name: K-Lox Proofer), and the free base nicotine (manufactured by Sigma) was directly applied to the adhesive surface of the crosslinked adhesive layer of laminate 2. The coating rate was constantly 0.1 m/min. Then, laminate 1 (polyester film release liner) was peeled off to expose an adhesive surface, which was adhered to the adhesive surface of the crosslinked adhesive layer of the above-mentioned laminate 2, which was coated with the free base nicotine to give nicotine transdermal delivery systems of Examples 1-5.

### (Examples 6-10)

In the same manner as in Example 1 except that Coconad MT (manufactured by Kao Corporation, caprylic • capric triglyceride) was used instead of isopropyl myristate, adhesive solution A was used in an amount corresponding to the adhesive solid content of 49.93, 54.923, 59.916, 64.909, 69.902 parts, Coconad MT was used in an amount of 50, 45, 40, 35 and 30 parts relative to the adhesive solid content, and Coronate HL (manufactured by Nippon Polyurethane Industry Co., Ltd.) was added in a proportion of 0.07, 0.077, 0.084, 0.091 and 0.098 parts, respectively (0.14% of adhesive), to give nicotine transdermal delivery systems of Examples 6-10.

### (Examples 11-14)

2-Ethylhexyl acrylate/vinyl acetate/2-hydroxyethyl acrylate=78/16/6 (weight ratio, DURO-TAK2196, manufactured by National Starch & Chemical Company) was measured out in the amounts corresponding to adhesive solid contents of 79.68, 69.72, 59.76 and 49.80 parts and each placed in a reaction container. Coconad MT (manufactured by Kao Corporation, caprylic • capric triglyceride) was added to each reaction container in a proportion of 20, 30, 40 and 50 parts relative to the adhesive solid content, ALCH (manufactured by Kawaken Fine Chemicals Co., Ltd., aluminum ethylacetoacetate • diisopropylate) as a crosslinking agent was added in a proportion of 0.32, 0.28, 0.24 and 0.20 parts (0.4% of adhesive) and the mixture was thoroughly stirred. The obtained solutions were each used for the preparation of the crosslinked adhesive layers of Examples 11-14 to be mentioned below.

The obtained solution was applied to a peel treated surface of a polyester film release liner having the peel treated surface on one side to a thickness after drying of 80 µm, and dried at 100°C for 3 min to give an adhesive layer. The adhesive surface of the adhesive layer thus formed was adhered to the surface on a non-woven fabric side of a support prepared by laminating a 2 µm thick polyethylene terephthalate film on the polyester non-woven fabric (fabric weight amount 12 g/m²) by extrusion forming to give a laminate. The obtained laminate was tightly sealed, left standing at 60°C for 48 hr to form a crosslinked adhesive layer. Thereafter, the release liner of the laminate was peeled off to expose the adhesive surface, an engraving roller (coated amount: calculated 1.75 mg/cm²) was set in a flexo printing coater (manufactured by RK Print Coat Instruments Ltd., product name: K-Lox Proofer), and a free base nicotine (manufactured by Sigma) was directly applied to the adhesive surface of the crosslinked adhesive layer. The coating rate was constantly 0.1 m/min. The adhesive layer was impregnated with the free base nicotine, and the adhesive surface was coated with a polyester film release liner to give nicotine transdermal delivery systems of Examples 11-14.

### (Example 15)

Under a nitrogen atmosphere, 2-ethylhexyl acrylate (72 parts), N-vinyl-2-pyrrolidone (25 parts), and acrylic acid (3 parts) were charged in a flask, and azobisisobutyronitrile (0.3 part) as a polymerization initiator was added to start polymerization. By adjusting the stirring rate and outer bath temperature, and dropwise addition of ethyl acetate, the inner bath temperature was adjusted to 58-62°C, and polymerization was carried out to give an adhesive solution.

The obtained adhesive solution was measured out in an amount corresponding to the adhesive solid content of 59.82 parts and placed in a reaction container. Coconad MT (manufactured by Kao Corporation, caprylic • capric triglyceride) was added to the reaction container in a proportion of 40 parts relative to the adhesive solid content, ALCH (manufactured by Kawaken Fine Chemicals Co., Ltd., aluminum ethylacetoacetate • diisopropylate) as a crosslinking agent was added in a proportion of 0.18 part (0.3% of adhesive) and the mixture was thoroughly stirred. The obtained solution was used for the preparation of the crosslinked adhesive layer of Example 15 to be mentioned below.

The obtained solution was applied to a peel treated surface of a polyester film release liner having the peel treated surface on one side to a thickness after drying of 40 µm, and dried at 100°C for 3 min to give an adhesive layer. The adhesive surface of the adhesive layer thus formed was adhered to the surface on a non-woven fabric side of a support prepared by laminating a 2 µm thick polyethylene terephthalate film on the polyester non-woven fabric (fabric weight amount 12 g/m²) by extrusion forming to give a laminate. The obtained laminate was tightly sealed, left standing at 60°C for 48 hr to form a crosslinked adhesive layer. Thereafter, the release liner of the laminate was peeled off to expose an adhesive surface, a bar coater No. 10 (film thickness: about 22.9 µm) was set in a flexo printing coater (manufactured by RK Print Coat Instruments Ltd., product name: K-Lox Proofer), and a free base nicotine was uniformly applied to a stainless plate with the bar coater. The adhesive surface of the crosslinked adhesive layer was adhered thereto and impregnated with a free base nicotine (1.75 mg/cm², manufactured by Sigma). Then, the polyester film release liner was adhered to the adhesive surface of the crosslinked adhesive layer, which had been coated with the free base nicotine, of the above-mentioned laminate to give a nicotine transdermal delivery system of Example 15.

### (Example 16)

In the same manner as in Example 15 except that the thickness of the adhesive layer after drying was 80 µm instead of 40 µm and the bar coaters used were No. 10 (film thickness: about 22.9 µm) and No. 3 (film thickness: about 6.87 µm), the No. 10 (film thickness: about 22.9 µm) bar coater was first used to impregnate a crosslinked adhesive layer with a free base nicotine (manufactured by Sigma), and the No. 3 (film thickness: about 6.87 µm) bar coater was used to impregnate the crosslinked adhesive layer with the free base nicotine (3.93 mg/cm², manufactured by Sigma), a nicotine transdermal delivery system of Example 16 was obtained.

### (Example 17)

In the same manner as in Example 16 except that the thickness of the adhesive layer after drying was 40 µm instead of 80 µm, a nicotine transdermal delivery system of Example 17 was obtained.

### (Example 18)

Under a nitrogen atmosphere, 2-ethylhexyl acrylate (72 parts), N-vinyl-2-pyrrolidone (25 parts) and acrylic acid (3 parts) were charged in a flask, and azobisisobutyronitrile (0.3 part) was added as a polymerization initiator to start polymerization. By adjusting the stirring rate and outer bath temperature, and dropwise addition of ethyl acetate, the inner bath temperature was adjusted to 58-62°C, and polymerization was carried out to give an adhesive solution.

The obtained adhesive solution was measured out in an amount corresponding to the adhesive solid content of 59.82 parts, isopropyl myristate was added in a proportion of 20 parts relative to the adhesive solid content, Coconad MT (manufactured by Kao Corporation, caprylic • capric triglyceride) was added in a proportion of 20 parts relative to the adhesive solid content and ALCH (manufactured by Kawaken Fine Chemicals Co., Ltd., aluminum ethylacetoacetate • diisopropylate) as a crosslinking agent was added in a proportion of 0.18 part (0.3% of adhesive) and the mixture was thoroughly stirred.

The obtained solution was applied to a peel treated surface of a polyethylene terephthalate release liner having the peel treated surface on one side to a thickness after drying of 60 µm, and dried at 70°C for 2 min and then 90°C for 2 min to give an adhesive layer. The adhesive surface was adhered to the surface on a non-woven fabric side of a support prepared by laminating a 2 µm thick polyethylene terephthalate film on the polyethylene terephthalate non-woven fabric (fabric weight amount 12 g/m²) by extrusion forming to give a laminate. The obtained laminate was tightly sealed, left standing at 60°C for 48 hr to form an adhesive layer.

Then, the release liner was peeled off while applying a free base nicotine to the adhesive layer at 1.8 g/cm² with a die coater. A polyethylene terephthalate release liner was adhered to the nicotine-coated surface to give a nicotine transdermal delivery system (width 100 mm, length 10 m). (Examples 19-22)

Under a nitrogen atmosphere, 2-ethylhexyl acrylate (72 parts), N-vinyl-2-pyrrolidone (25 parts), and acrylic acid (3 parts) were charged in a flask, and azobisisobutyronitrile (0.3 part) as a polymerization initiator was added to start polymerization. By adjusting the stirring rate and outer bath temperature, and dropwise addition of ethyl acetate, the inner bath temperature was adjusted to 58-62°C, and polymerization was carried out to give an adhesive solution.

The obtained adhesive solution was measured out in amounts corresponding to the adhesive solid content of 59.82 parts and placed in respective reaction containers. Isopropyl myristate and/or Coconad MT (manufactured by Kao Corporation, caprylic • capric triglyceride) were/was added to the reaction containers at isopropyl myristate (40 parts, Example 19), isopropyl myristate (20 parts) and Coconad MT (20 parts, Example 20), isopropyl myristate (10 parts) and Coconad MT (30 parts, Example 21), and Coconad MT (40 parts, Example 22), respectively, relative to the adhesive solid content, ALCH (manufactured by Kawaken Fine Chemicals Co., Ltd., aluminum ethylacetoacetate • diisopropylate) as a crosslinking agent was added in a proportion of 0.18 part (0.3% of adhesive) and the mixtures were thoroughly stirred. The obtained solutions were used for the preparation of the crosslinked adhesive layers of Example 19-22 to be mentioned below.

The obtained solutions were applied to a peel treated surface of polyester film release liners having the peel treated surface on one side, to a thickness after drying of 80 µm, and dried at 100°C for 3 min to give an adhesive layer. The adhesive surface of the adhesive layer thus formed was adhered to the surface on a non-woven fabric side of a support prepared by laminating a 2 µm thick polyethylene terephthalate film on the polyester non-woven fabric (fabric weight amount 12 g/m²) by extrusion forming to give a laminate. The obtained laminate was tightly sealed, left standing at 60°C for 48 hr to form a crosslinked adhesive layer. Thereafter, the release liner of the laminate was peeled off to expose an adhesive surface, a bar coater No. 10 (film thickness: about 22.9 µm) was set in a flexo printing coater (manufactured by RK Print Coat Instruments Ltd., product name: K-Lox Proofer), and a free base nicotine was uniformly applied to a stainless plate with the bar coater. The adhesive surface of the crosslinked adhesive layer was adhered thereto and impregnated with a free base nicotine (1.8 mg/cm², manufactured by Sigma). Then, the polyester film release liner was adhered to the adhesive surface of the crosslinked adhesive layer, which had been coated with the free base nicotine, of the above-mentioned laminate to give nicotine transdermal delivery systems of Examples 19-22.

### (Comparative Example 1)

In the same manner as in Example 1 except that the amount of the adhesive solution A based on the adhesive solid content was set to 89.874 parts, isopropyl myristate was set to 10 parts and Coronate HL was set to 0.126 part, a nicotine transdermal delivery system was obtained.

### (Comparative Example 2)

In the same manner as in Comparative Example 1 except that Coconad MT (manufactured by Kao Corporation, caprylic · capric triglyceride) was used instead of isopropyl myristate, a nicotine transdermal delivery system was obtained.

### (Comparative Example 3)

In the same manner as in Comparative Example 1 except that isopropyl myristate was set to 80 parts instead of 10 parts, the amount of the adhesive solution A based on the adhesive solid content was set to 19.97 parts instead of 89.874 parts, and Coronate HL was set to 0.03 part instead of 0.126 part, a nicotine transdermal delivery system was obtained.

### (Experimental Example 1) [Evaluation of preparations]

The following evaluation was made with regard to nicotine transdermal delivery systems (samples) obtained in Examples 1-10 and Comparative Examples 1 and 2.

### Evaluation of adhesiveness, adhesion

The samples were cut into a width of 24 mm and a length of 80 mm, and quickly adhered to a phenol resin test board having a width of 30 mm, a length of 150 mm, and a thickness of 2 mm, with one end of the samples matched to one end of the board. A rubber roller weighing 850 g was immediately passed twice on the samples at 300 mm per minute. The samples were left standing at 23±2°C for 30 min. About 5 mm of the adhered one end of the sample was detached and folded back 180°. Auxiliary paper was adhered to extend the length and, using a tensile tester (EZTest, manufactured by Shimadzu Corporation), one end of the auxiliary paper was held tight with a catch at an upper part of the tester, the test board was held tight with a catch at a lower part of the tester, the samples were continuously peeled off at 300 mm per minute, and the weight was measured.

### Evaluation of pain upon peeling off

The samples formed into 10 cm² were adhered to the upper arm of six healthy volunteers for 24 hr and the pain upon peeling off of the sample from the skin was evaluated according to 5 levels of evaluation scores.

| | |
|---|---|
| 1: not painful | 2: only slightly painful |
| 3: slightly painful | 4: a little painful |
| 5: very painful | |

### Evaluation of easy taking out from package

The sample was punched out in 20 cm², and tightly sealed in a pouch (5 cm×5 cm) made of a laminate film of polyethylene/aluminum/polyacrylonitrile resin. Then, the sample was preserved at room temperature and at 40°C, and easy taking out of the sample from the package was observed.

For the evaluation of easy taking out from package, when the sample could be taken out from the package without any problem, "not problem" was indicated, and when the adhesive oozed through the edge of the sample to cause attachment of the sample to the inside of the pouch, making easy taking out from the package difficult to achieve, the condition was described.

The results of adhesiveness evaluation, evaluation of pain upon peeling off and evaluation of easy taking out from package are shown in Table 1. From the results of Table 1, the nicotine transdermal delivery systems of Examples 1-10 were shown to be superior in the fixedness and a soft feeling during application, because of the addition of a liquid ingredient compatible with the adhesive at a content ratio in a particular range.

**Table 1**

| | liquid ingredient | | adhesion (N=3) N/24 mm | pain upon peeling off | falling off of sample in 6 volunteers during adhesion for 24 hr | easy taking out from package |
|---|---|---|---|---|---|---|
| | name | parts by weight relative to whole adhesive layer (100 parts by weight) | average | average score | Number of volunteers with falling off | |
| Ex. 1 | isopropyl myristate | 50 | 1.7 | 1.0 | 0/6 | n.p. |
| Ex. 2 | isopropyl myristate | 45 | 1.9 | 1.1 | 0/6 | n.p. |
| Ex. 3 | isopropyl myristate | 40 | 2.0 | 1.0 | 0/6 | n.p. |
| Ex. 4 | isopropyl myristate | 35 | 2.4 | 1.5 | 0/6 | n.p. |
| Ex. 5 | isopropyl myristate | 30 | 2.8 | 1.6 | 0/6 | n.p. |
| Ex. 6 | Coconad MT | 50 | 3.3 | 1.2 | 0/6 | n.p. |
| Ex. 7 | Coconad MT | 45 | 3.0 | 1.2 | 0/6 | n.p. |
| Ex. 8 | Coconad MT | 40 | 3.3 | 1.3 | 0/6 | n. p. |
| Ex. 9 | Coconad MT | 35 | 2.6 | 1.6 | 0/6 | n.p. |
| Ex. 10 | Coconad MT | 30 | 3.0 | 1.5 | 0/6 | n.p. |
| Comp. Ex. 1 | isopropyl myristate | 10 | 3.6 | 4.7 | 0/6 | n.p. |
| Comp. Ex. 2 | Coconad MT | 10 | 3.3 | 4.3 | 0/6 | n.p. |
| Comp. Ex. 3 | isopropyl myristate | 80 | _* | _* | _* | _* |

| | | | | | | |
|---|---|---|---|---|---|---|
| n.p. no problem * Production was not possible because of too much liquid ingredient that prevented sufficient cohesion. | | | | | | |

### (Experimental Example 2) Quantitation of nicotine content

The nicotine transdermal delivery systems of Examples 1-22 were formed into 10 cm², extracted with methanol, the free base nicotine content of the extract was quantitated using reversed-phase HPLC, and a free base nicotine content per 1 cm² of the preparation (average of 3 cases) and a free base nicotine content rate of the adhesive layer (average of 3 cases) were calculated. The results are shown in Table 2. In the Table, SD shows standard deviation.

**Table 2**

| | liquid ingredient | | free base nicotine content | | free base nicotine nicotine content rate | |
|---|---|---|---|---|---|---|
| | name | parts by weight relative to whole adhesive layer (100 parts by weight) | (mg/cm²) | SD | (%) | SD |
| Ex. 1 | isopropyl myristate | 50 | 1.819 | 0.052 | 5.99 | 0.19 |
| Ex. 2 | isopropyl myristate | 45 | 1.794 | 0.045 | 6.10 | 0.32 |
| Ex. 3 | isopropyl myristate | 40 | 1.834 | 0.026 | 6.66 | 0.33 |
| Ex. 4 | isopropyl myristate | 35 | 1.819 | 0.066 | 6.70 | 0.19 |
| Ex. 5 | isopropyl myristate | 30 | 1.796 | 0.070 | 6.68 | 0.31 |
| Ex. 6 | Coconad MT | 50 | 1.776 | 0.011 | 6.15 | 0.42 |
| Ex. 7 | Coconad MT | 45 | 1.832 | 0.038 | 6.08 | 0.24 |
| Ex. 8 | Coconad MT | 40 | 1. 859 | 0.019 | 6.37 | 0.42 |
| Ex. 9 | Coconad MT | 35 | 1.765 | 0.064 | 6.29 | 0.29 |
| Ex. 10 | Coconad MT | 30 | 1.724 | 0.060 | 6.09 | 0.31 |
| Ex. 11 | Coconad MT | 20 | 1.80 | 0.02 | 13.14 | 0.24 |
| Ex. 12 | Coconad MT | 30 | 1.79 | 0.02 | 12.39 | 0.46 |
| Ex. 13 | Coconad MT | 40 | 1.85 | 0.02 | 13.86 | 0.32 |
| Ex. 14 | Coconad MT | 50 | 1.81 | 0.06 | 13.37 | 0.50 |
| Ex. 15 | Coconad MT | 40 | 1.80 | 0.02 | 31.27 | 0.34 |
| Ex. 16 | Coconad MT | 40 | 3.00 | 0.13 | 25.68 | 0.87 |
| Ex. 17 | Coconad MT | 40 | 3.81 | 0.17 | 30.58 | 0.95 |
| Ex. 18 | isopropyl myristate Coconad MT | 40 | 2.09 | 0.058 | 24.71 | 0.75 |
| Ex. 19 | isopropyl myristate | 40 | 1.84 | 0.01 | 18.58 | 0.10 |
| Ex. 20 | isopropyl myristate Coconad MT | 40 | 1.87 | 0.10 | 18.87 | 0.81 |
| Ex. 21 | isopropyl myristate Coconad MT | 40 | 1.81 | 0.08 | 17.88 | 0.67 |
| Ex. 22 | Coconad MT | 50 | 1.83 | 0.02 | 17.40 | 0.44 |

In the Table, the parts by weight (40 parts) per 100 parts of the whole adhesive layers in Examples 18 and 20 is a total of isopropyl myristate (20 parts) and Coconad MT (20 parts) and the parts by weight (40 parts) per 100 parts of the whole adhesive layer in Example 21 is a total of isopropyl myristate (10 parts) and Coconad MT (30 parts).

### (Experimental Examples 3-6)

The shed snakeskin permeability test and hairless mouse skin permeability test in the following Experimental Examples 3-6 were conducted according to the following method.

### [Shed snakeskin permeability test]

permeation apparatus: full automatic flow through diffusion cell apparatus (manufactured by VANGUARD INTERNATIONAL) sample area: 0.2826 cm²
receptor solution: phosphate buffer (pH=7.4), containing 0.02% sodium azide
flow: about 10 mL/4 hr/cell (number of rotations of pump: 20 rpm)

A preparation (sample) was adhered to the center of the outside (smooth surface side) of shed snakeskin (African Rock Python) sufficiently hydrated and punched into a size of 2.5434 cm², and set in a permeation cell. A receptor solution kept at 32°C was flown to activate a fraction collector to start the experiment. The receptor solution was recovered every 1, 2, 3, 4, 5, 6, 7, 8, 12, 16, 20, 24 hr. The free base nicotine content of the recovered receptor solution was quantitated by HPLC. The unit time (h), drug permeation amount (µg/cm²/h) per unit area (cm²), and cumulative permeation amount (µg/cm²) per unit area were calculated.

### [hairless mouse skin permeability test]

permeation apparatus: upright PERMCELL TP-20 (manufactured by Vidrex Company Limited)
sample area: 2.0096 cm²
receptor solution: phosphate buffer (pH=7.4), containing 0.02% sodium azide

A preparation (sample) was adhered to the center of the hairless mouse skin and immediately set in a permeation cell. A receptor solution (ca. 20 ml) warmed to 32°C was placed in the cell without including air foams. A refluxing solution cell at 32°C was supplied from a thermostatic tank to a jacket of the cell, and vigorously stirred with a stirrer in the cell under an atmosphere at 32°C. The receptor solution in the cell was all recovered every 20 min, 40 min, 60 min, 100 min, 2 hr, 3 hr, 4 hr, 5 hr, 6 hr, 7 hr, 8 hr, 24 hr, and a fresh receptor solution (20 ml) heated to 32°C was added to the cell. The free base nicotine content of the recovered receptor solution was quantitated by HPLC. The unit time (h), drug permeation amount (µg/cm²/h) per unit area (cm²) and cumulative permeation amount (µg/cm²) per unit area were calculated. (Experimental Example 3) Permeability test

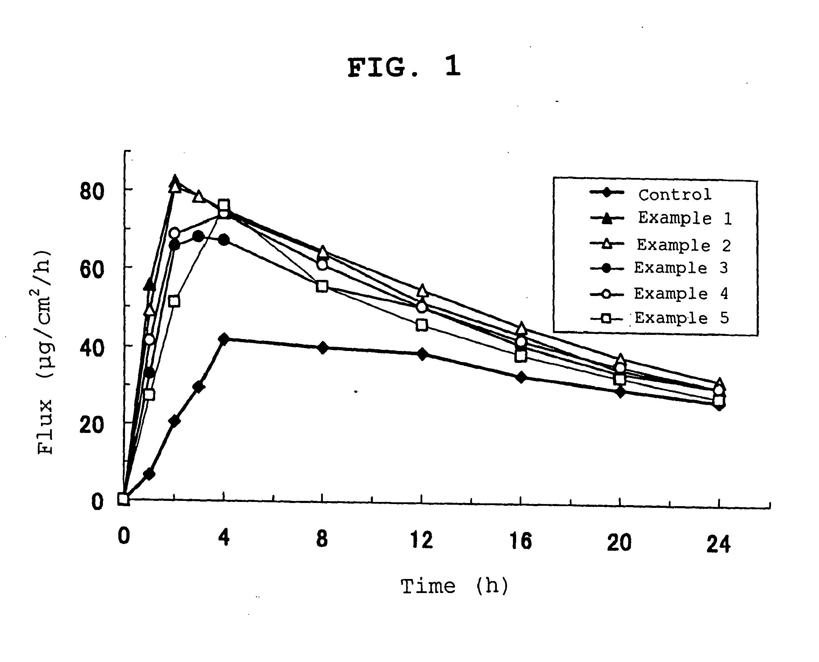

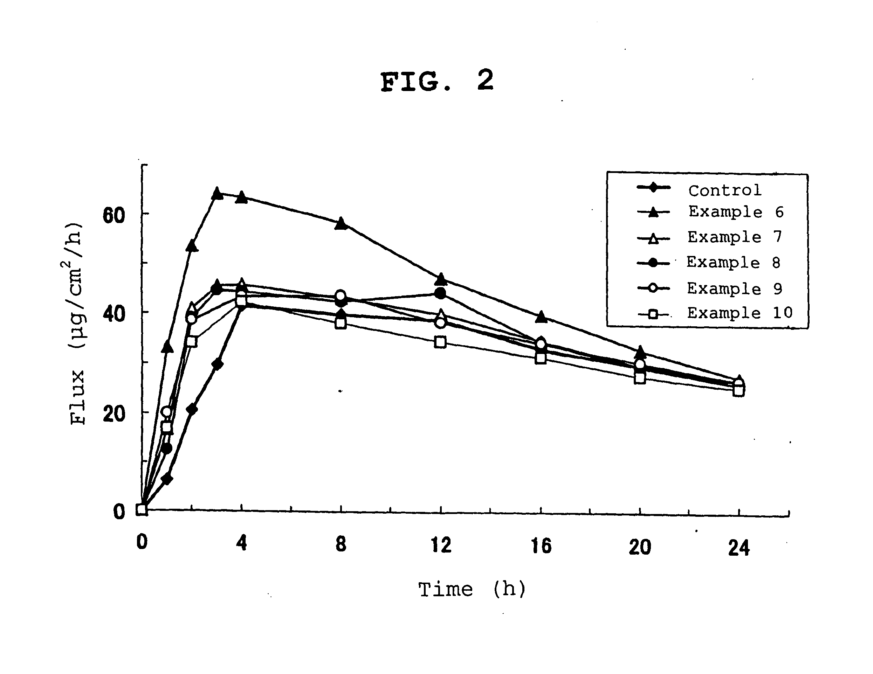

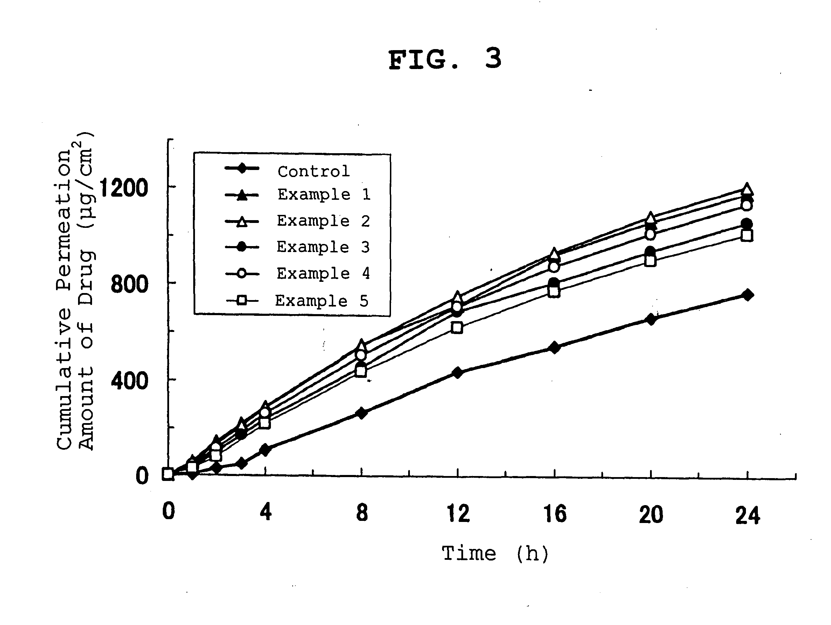

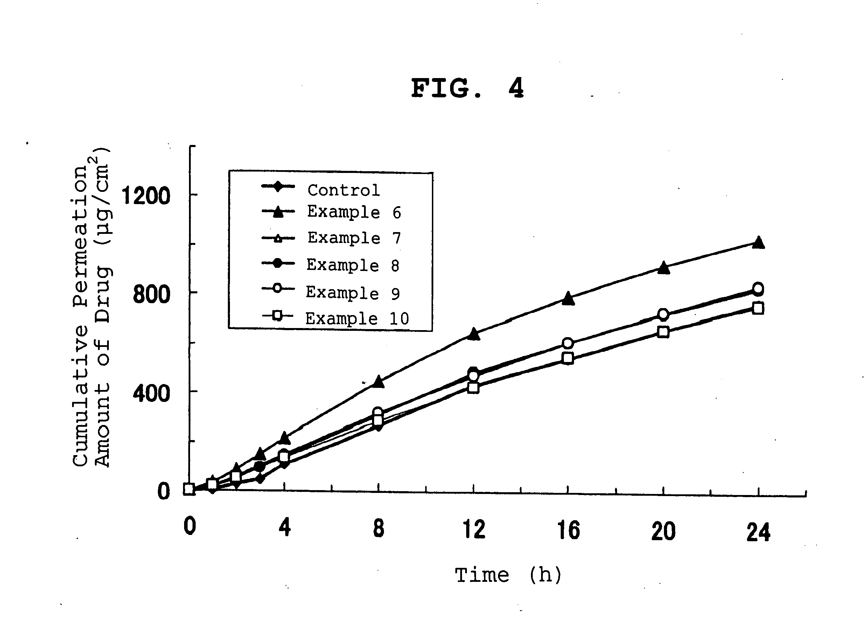

The drug permeability of nicotine transdermal delivery systems (samples) of Examples 1-10 was evaluated using shed snakeskin (African Rock Python). As a control, a nicotine transdermal delivery system Nicotinell TTS (manufactured by Novartis Pharma K.K.) was used. The results are shown in Figs. 1-4. The results are average values of 3 measures. (Experimental Example 4) Permeability test

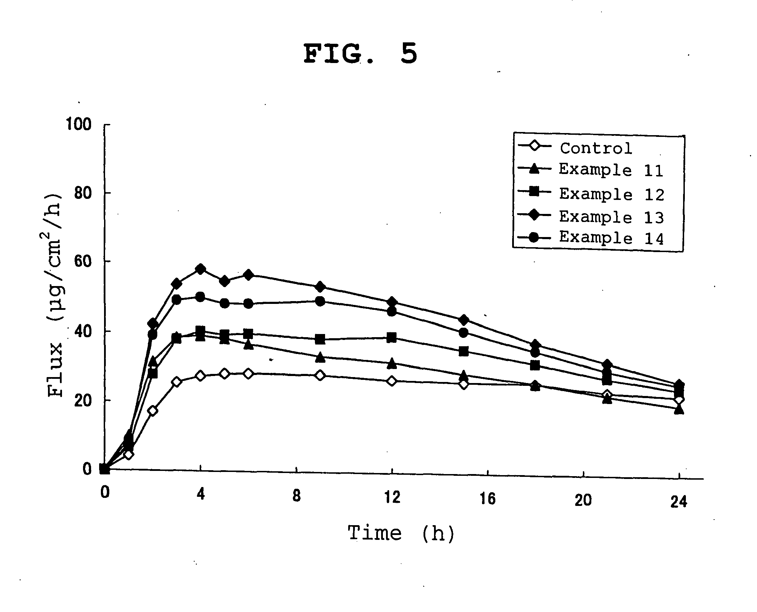

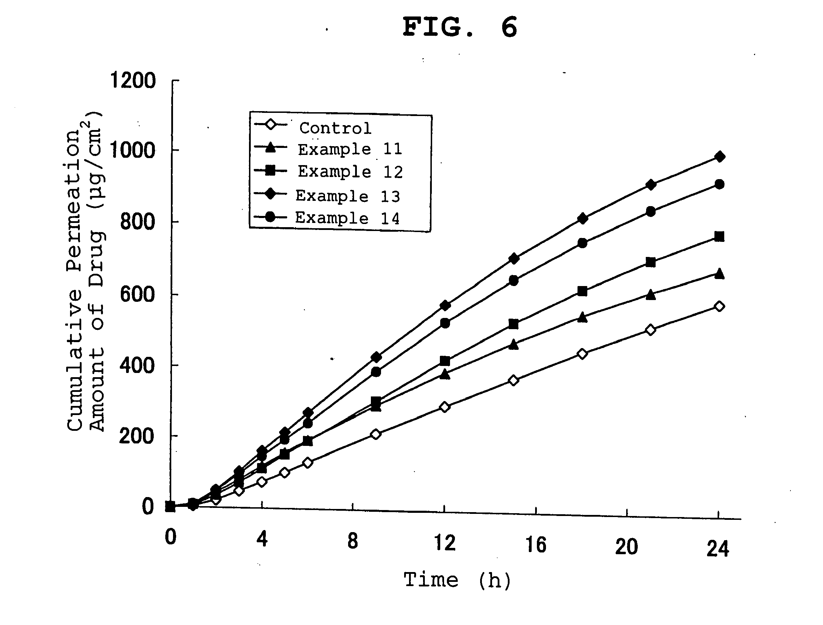

The drug permeability of nicotine transdermal delivery systems (samples) of Examples 11-14 was evaluated using a skin removed from a hairless mouse. As a control, a nicotine transdermal delivery system Nicotinell TTS (manufactured by Novartis Pharma K.K.) was used. The results are shown in Figs. 5-6. The results are average values of 6 measures. (Experimental Example 5) Permeability test

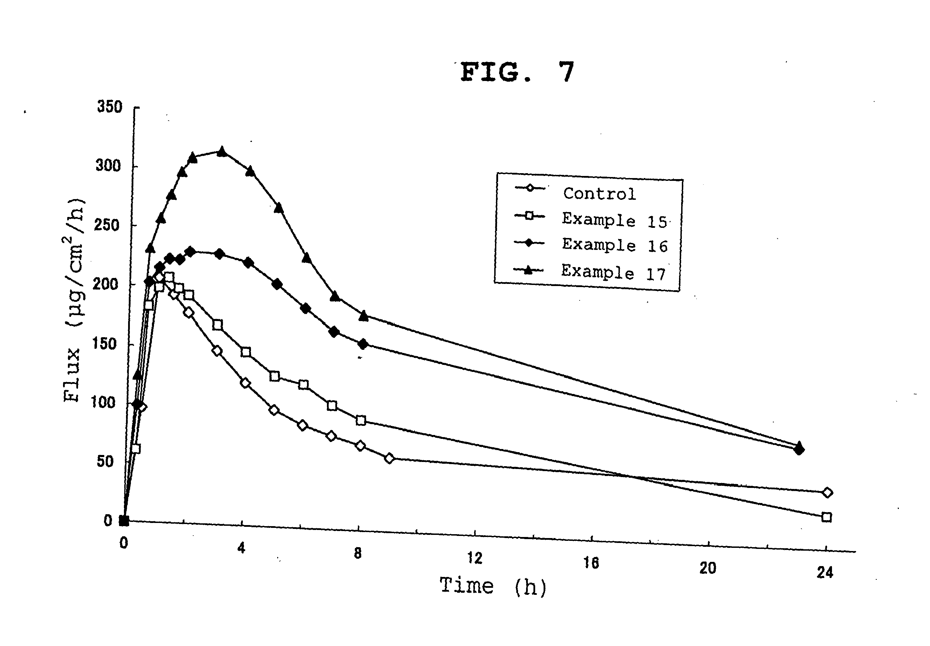

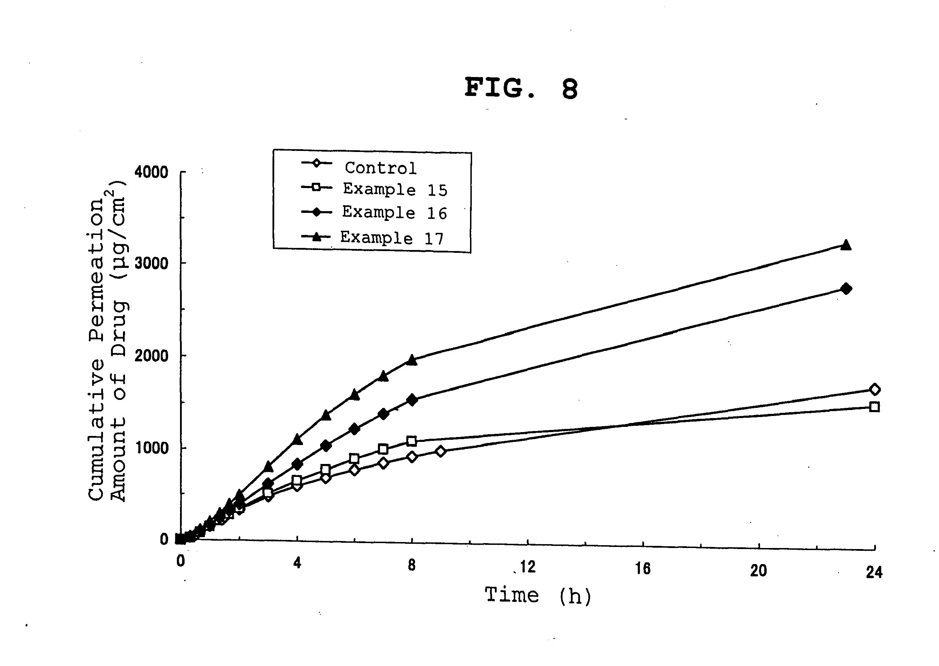

The drug permeability of nicotine transdermal delivery systems (samples) of Examples 15-17 was evaluated using a skin removed from a hairless mouse. As a control, a nicotine transdermal delivery system (NICODERM CQ CLEAR, manufactured by ALZA Corporation) was used. The results are shown in Figs. 7-8. The results are average values of 3 measures. (Experimental Example 6) Permeability test

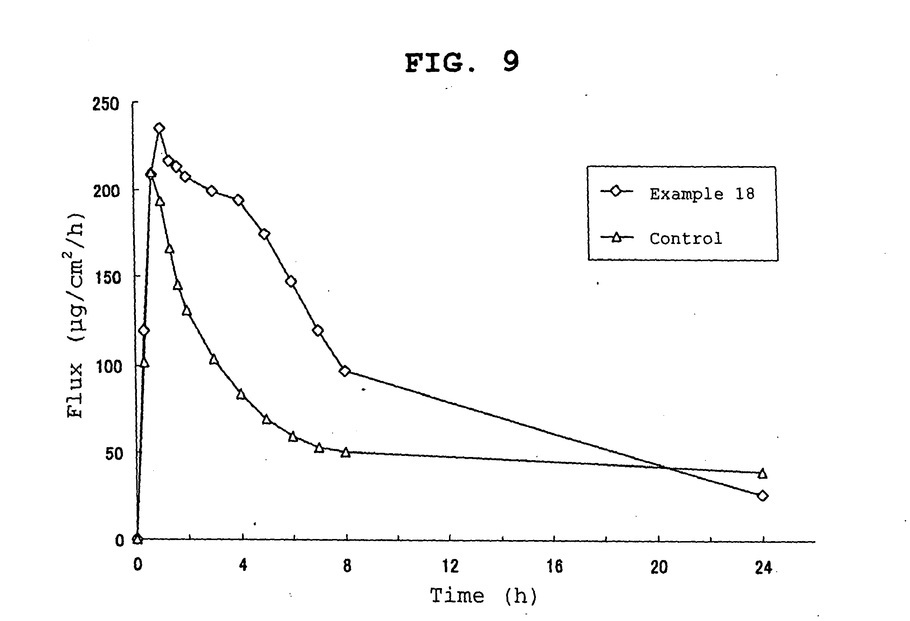

The drug permeability of a nicotine transdermal delivery system (sample) of Examples 18-22 was evaluated using a skin removed from a hairless mouse (intact). In Examples 18, a nicotine transdermal delivery system (NICODERM CQ CLEAR, manufactured by ALZA Corporation) was used as a control (Fig. 9). The results are shown in Figs. 9-11. The results are average values of 3 measures.

As shown above, the nicotine transdermal delivery system of the present invention shows extremely superior skin adhesion characteristic and remarkably small pain upon peeling off, as compared to the preparations of Comparative Examples. Such characteristic is preferable for a nicotine transdermal delivery system to be adhered every day. In addition, the risk of falling off during use is small and the preparation is highly economical.

In a nicotine permeability test, moreover, the preparation of the present invention showed permeability equal to or not less than that of control preparations of Nicotinell TTS and NICODERM CQ CLEAR.

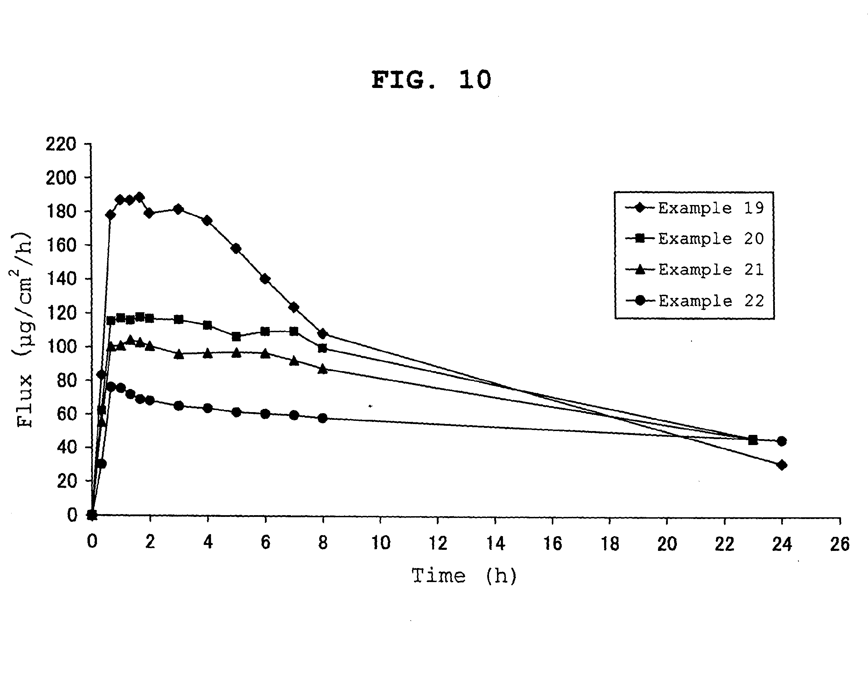

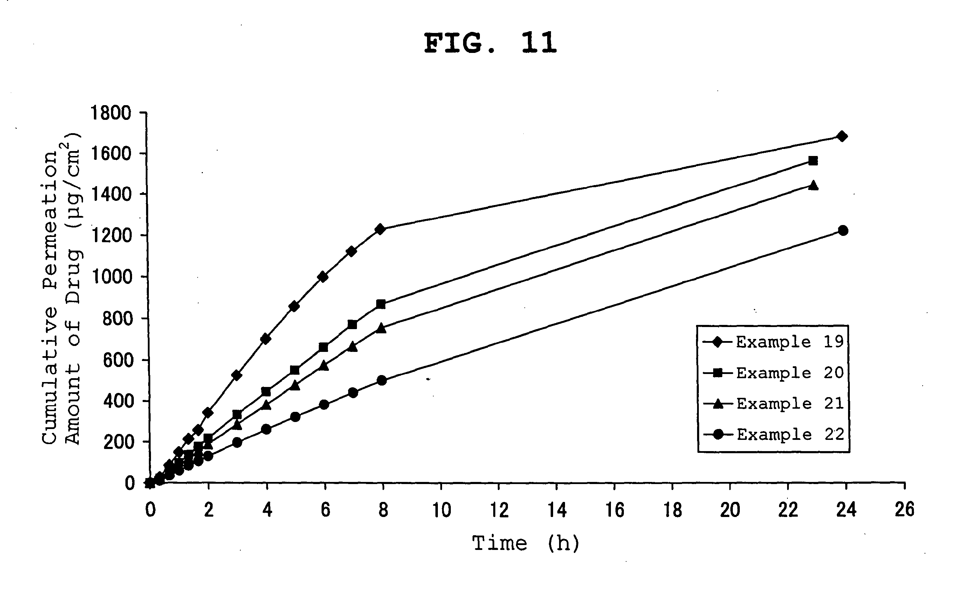

Furthermore, as is clear from the results of Figs. 10 and 11, the use of a coexistent system of isopropyl myristate and caprylic · capric triglyceride (Example 20 and Example 21) was shown to particularly afford appropriate transdermal absorbability, which was not too high or too low. This means that the coexistent system of isopropyl myristate and caprylic · capric triglyceride is advantageous in a certain kind of application, for example, a one time/day adhesion program.

### INDUSTRIAL APPLICABILITY

The nicotine transdermal delivery system of the present invention shows good adhesiveness and cohesion, and simultaneously achieves low irritation to the skin during peeling off and a fine feeling during adhesion. Therefore, the preparation can be used for those wishing to quit smoking, particularly, following the stop-smoking program currently being practiced.

## Claims

1. A nicotine transdermal delivery system comprising an adhesive layer comprising a free base nicotine and a liquid ingredient compatible with the adhesive, wherein the adhesive layer is crosslinked, and the liquid ingredient is contained in a proportion of 20-75 parts by weight, per 100 parts by weight of the adhesive layer as a whole.

2. A nicotine transdermal delivery system according to claim 1, wherein the liquid ingredient is a fatty acid alkyl ester and/or glycerol fatty acid ester.

3. A nicotine transdermal delivery system according to claim 1 or claim 2, wherein the liquid ingredient is contained in a proportion of 20-60 parts by weight, per 100 parts by weight of the adhesive layer as a whole.

4. A nicotine transdermal delivery system according to any one of the preceding claims, wherein the aforementioned liquid ingredient is isopropyl myristate and/or caprylic/capric triglyceride.

5. A nicotine transdermal delivery system according to any one of the preceding claims, wherein the adhesive contained in the adhesive layer is an acrylic adhesive.

6. A nicotine transdermal delivery system according to claim 5, wherein the acrylic adhesive comprises (meth)acrylic acid alkyl ester and a vinyl monomer having a functional group capable of being involved in a crosslinking reaction in a weight ratio of 40:10 to 99.9-0.1.

7. A nicotine transdermal delivery system according to claim 6, wherein the (meth)acrylic acid alkyl ester is 2-ethylhexyl acrylate and the vinyl monomer having a functional group capable of being involved in a crosslinking reaction is acrylic acid and/or 2-hydroxyethyl acrylate.

8. A nicotine transdermal delivery system according to any one of the preceding claims, wherein the adhesive layer comprises the free base nicotine in a proportion of 1-40 wt%.

9. A nicotine transdermal delivery system according to any one of the preceding claims, for use in a stop-smoking program.

10. Use of an adhesive layer as defined in any one of claims 1 to 8, in the manufacture of a medicament for use in a stop-smoking program.
